# EUROPEAN PATENT SPECIFICATION

(11) **EP 1 050 301 B1**
(45) Date of publication and mention of the grant of the patent: **25.03.2009**
(21) Application number: 99900351.0
(22) Date of filing: 19.01.1999
(51) Int. Cl.: A61K 31/135, A61K 47/40, A61K 47/26, A61K 9/08

(54) **Medicinal compositions containing 2-amino-2-[2-(4-octylphenyl)ethyl]propane-1,3-diol**
Medizinische Zusammensetzungen enthaltend 2-Amino-2-[2-(4-octylphenyl)ethyl]propan-1,3-diol
Compositions médicinales contenant 2-amino-2-[2-(4-octylphényl)éthyl]propane-1,3-diol

(30) Priority: 19.01.1998 JP 804598
(43) Date of publication of application: 08.11.2000
(73) Proprietor: Mitsubishi Tanabe Pharma Corporation, Chuo-ku Osaka-shi Osaka 541-8505 (JP)
(72) Inventor: SAKAI, Atsushi, c/o Welfide Corporation, Chikujo-gun, Fukuoka 871-8550 (JP); MASUDA, Rumiko, c/o Welfide Corporation, Chikujo-gun, Fukuoka 871-8550 (JP); FUJII, Tsuneo, c/o Welfide Corporation, Chikujo-gun, Fukuoka 871-8550 (JP); MISHINA, Tadashi, c/o Welfide Corporation, Hirakata-shi, Osaka 573-1153 (JP); CHIBA, Kenji, c/o Welfide Corporation, Iruma-shi, Saitama 358-0026 (JP)
(74) Representative: von Kreisler, Alek
(86) International application number: PCT/JP1999/000152
(87) International publication number: WO 1999/036065

(56) References cited:
- EP-A2- 0 335 545
- WO-A1-94/08943
- WO-A1-96/06068
- WO-A1-98/03162
- JP-A- 5 213 757
- JP-A- 6 016 547
- JP-A- 7 228 532
- JP-A- 7 316 065
- JP-A- 8 175 985
- JP-A- 58 148 816
- JP-A- 63 253 022

## Description

### Technical Field

The present invention relates to a pharmaceutical composition containing 2-amino-2-[2-(4-octylphenyl)ethyl]propane-1,3-diol or a pharmaceutically acceptable acid addition salt thereof as an active ingredient and to a composition for a kit. More particularly, the present invention relates to a pharmaceutical composition containing 2-amino-2-[2-(4-octylphenyl)ethyl]propane-1,3-diol or a pharmaceutically acceptable acid addition salt thereof and cyclodextrin as a stabilizer, which is suitable for the suppression of rejection in organ (e.g., kidney, liver, heart, small bowel etc.) or bone marrow transplantation, immunosuppressive sustention therapy therefor and the treatment of autoimmune diseases, and which can be formulated into a liquid agent.

### Background Art

2-Amino-2-[2-(4-octylphenyl)ethyl]propane-1,3-diol and a pharmaceutically acceptable acid addition salt thereof are known to be useful as suppressants of rejection in organ or bone marrow transplantation and as therapeutic agents for various autoimmune diseases, such as psoriasis, Behçet's disease and the like, and rheumatic diseases, as described in WO-A-94/08943.

While this compound has been developed as a preparation for oral administration, when it is used as a suppressant of rejection in organ or bone marrow transplantation, the administration thereof immediately after transplantation is desired for the quickest possible exertion of the effect. In view of the condition of patient, however, since oral administration is difficult for the patient, it is administered by injection. When this compound is used for diseases of the eye, such as Behçet's disease, moreover, it needs to be applied as an eye drop.

The above-mentioned WO-A-94/08943 describes a preparation of this compound as an injection, and discloses the use of polyethylene glycol and ethanol as solubilizers. However, polyethylene glycol shows undesirable effects such as local irritation and hemolysis and its use is problematic. Ethanol is neither suitable for an injection because it causes local irritation.

WO-A-97/24112 discloses, as an external agent of this compound, an eye drop containing this compound and, as a solubilizer, hydrogenated polyoxyethylene castor oil, which is a surfactant.

When the above-mentioned compound, particularly 2-amino-2-[2-(4-octylphenyl)ethyl]propane-1,3-diol hydrochloride (hereinafter to be also referred to as the present compound throughout the specification), is dissolved in distilled water, the problems occur that the aqueous solution shows hemolytic property, foams due to the surface-activating property that the compound itself has, and shows precipitation of the present compound in the aqueous solution as crystals at a certain concentration due to the dissolution mechanism peculiar to the present compound, despite the fact that the present compound is water-soluble. In addition, an aqueous solution of the present compound incorporating an isotonicity agent (e.g., sodium chloride etc.) and/or a solubilizer (e.g., hydrogenated polyoxyethylene castor oil etc.) or a tackifier (e.g., polyvinylpyrrolidone etc.) as additives, that are generally used for liquid agents such as injection and eye drop, is associated with the above-mentioned problems of hemolysis, foaming and crystal precipitation, and none of the above are satisfactory.

JP-A-7-316065 discloses an FR901469 preparation containing cyclodextrin, which shows decreased hemolytic property and less local irritation, and JP-A-7-228532 discloses an aqueous liquid agent containing cyclodextrin, which shows enhanced water solubility and enhanced stability in water of a hardly water soluble agent. JP-A-133960/1976 discloses a method of eliminating foams produced by a surfactant for industrial use, which comprises adding cyclodextrin to a foamed aqueous solution containing the surfactant.

EP-A-335545 discloses a parenteral drug in an aqueous solution for injection to which beta- or gamma-cyclodextrin is added to prevent precipitation.

JP-A-63-253022 discloses that cyclodextrins give high solubility to an aqueous solution of baclofen.

### Disclosure of the Invention

In view of the above situation, the present inventors have conducted intensive studies in an attempt to obtain a pharmaceutical composition containing 2-amino-2-[2-(4-octylphenyl)ethyl]propane-1,3-diol or a pharmaceutically acceptable acid addition salt thereof, which is associated with less side effects, such as hemolysis, or local irritation, which is superior in defoaming performance, and which can be formulated into a liquid agent, such as an injection and an eye drop, that is free from crystal precipitation of the active ingredient compound, and found that the incorporation of cyclodextrin can achieve such obj ect, which resulted in the completion of the present invention.

Accordingly, the present invention provides a pharmaceutical composition containing 2-amino-2-[2-(4-octylphenyl)ethyl]propane-1,3-diol or a pharmaceutically acceptable acid addition salt thereof and cyclodextrin, which can be readily prepared into a pharmaceutical agent, which is associated with less side effects, such as hemolysis, or local irritation, which is superior in defoaming performance, and which is suitable for a liquid agent free from crystal precipitation of the active ingredient compound. The present invention characteristically resolves all the aforementioned problems simultaneously by the incorporation of cyclodextrin as a stabilizer along with 2-amino-2-[2-(4-octylphenyl)ethyl]propane-1,3-diol or a pharmaceutically acceptable acid addition salt thereof, and is particularly characterized in that it provides a liquid agent superior in defoaming performance and free from crystal precipitation of the active ingredient compound. In the present invention, it has been also found that the incorporation of a saccharide selected from monosaccharides, disaccharides and sugar alcohols results in a liquid composition further improved in local irritation.

The pharmaceutical composition of the present invention contains 2-amino-2-[2-(4-octylphenyl)ethyl]propane-1,3-diol or a pharmaceutically acceptable acid addition salt thereof as an active ingredient and cyclodextrin as a stabilizer, and where desired, a saccharide.

Cyclodextrin is used for the preparation of a pharmaceutical composition containing cyclodextrin, wherein crystal precipitation of the active ingredient compound has been inhibited, as an inhibitor of crystal precipitation of the active ingredient compound in a pharmaceutical composition containing cyclodextrin, and as a medical agent wherein crystal precipitation of the active ingredient compound in a pharmaceutical composition has been inhibited.

The 2-amino-2-[2-(4-octylphenyl)ethyl]propane-1,3-diol and a pharmaceutically acceptable acid addition salt thereof, that are the active ingredients of the pharmaceutical composition of the present invention, can be produced according to the method described in WO-A-94/08943. Preferred compound is 2-amino-2-[2-(4-octylphenyl)ethyl]propane-1,3-diol hydrochloride. As other acid addition salts, there are exemplified hydrobromide, sulfate, acetate, fumarate, maleate, benzoate, citrate, malate, methanesulfonate, benzenesulfonate and the like.

The 2-amino-2-[2-(4-octylphenyl)ethyl]propane-1,3-diol or a pharmaceutically acceptable acid addition salt thereof is added in a proportion of 0.01 - 20 wt%, particularly preferably 0.1 - 10 wt%, of the total weight of the composition.

The cyclodextrin to be used in the present invention is naturally occurring cyclodextrin, branched cyclodextrin, alkyl cyclodextrin or hydroxyalkyl cyclodextrin. Specifically exemplified are α-cyclodextrin (e.g., trademark: Cerdex A-100 manufactured by Nihon Shokuhin Kako Co., Ltd.), β-cyclodextrin (e.g., trademark: Cerdex B-100 manufactured by Nihon Shokuhin Kako Co., Ltd.), γ-cyclodextrin (e.g., trademark: Cerdex G-100 manufactured by Nihon Shokuhin Kako Co., Ltd.), dodecakis-2,6-O-methyl-α-cyclodextrin, tetradecakis-2,6-O-methyl-β-cyclodextrin, hexadecakis-2,6-O-methyl-γ-cyclodextrin, tetradecakis-2,6-O-ethyl-β-cyclodextrin, α-cyclodextrin partially etherified with 2-hydroxypropyl, β-cyclodextrin partially etherified with 2-hydroxypropyl (HP-β-CyD, e.g., trademark: Cerdex HP-β-CD manufactured by Nihon Shokuhin Kako Co., Ltd.), branched α-cyclodextrin and branched β-cyclodextrin wherein glucose and maltose have been bonded via α-1,6-glucoside bond, and the like. These cyclodextrins are added in an amount of 1-50 parts by weight, particularly preferably 10-30 parts by weight, per part by weight of the above-mentioned active ingredient.

The saccharide to be used in the present invention is selected from monosaccharides, disaccharides and sugar alcohols, and is exemplified by glucose, fructose, D-maltose, lactose, sucrose, D-mannitol, D-xylitol and D-sorbitol, which may be used alone or in combination. These saccharides are added in an amount of 1-100 parts by weight, particularly preferably 5-80 parts by weight, per part by weight of 2-amino-2-[2-(4-octylphenyl)ethyl]propane-1,3-diol or a pharmaceutically acceptable acid addition salt thereof.

When 2-amino-2-[2-(4-octylphenyl)ethyl]propane-1,3-diol or a pharmaceutically acceptable acid addition salt thereof (particularly hydrochloride) is prepared into an aqueous solution, it foams at a concentration of 0.01 - 20 wt%, and causes hemolysis and local irritation. The present compound shows special dissolution mechanism wherein, when it is prepared into an aqueous solution having a concentration higher than 0.1 wt%, it is dissolved after forming micelle, and at a concentration lower than 0.05 wt%, it is dissolved to give a solution without forming micelle. As a result of this unique dissolution mechanism, crystals are precipitated in an aqueous solution having a concentration of 0.05 - 0.1 wt%. The incorporation of cyclodextrin into the aqueous solution enables inhibition of the precipitation of crystals due to the formation of micelle, as well as simultaneous resolution of the problems of foaming, hemolysis and local irritation, which characterizes the present invention.

The pharmaceutical composition of the present invention takes the preparation form of a liquid agent, which may be injection, eye drop, nose drop, ear drop, infusion, liquid for oral administration, liquid for inhalation, liquid for lotion and the like, with preference given to injection (intravenous, subcutaneous, intramuscular, etc.) eye drop and infusion. These preparation forms are appropriately selected according to the diseases to be treated, conditions thereof, sex and age of patient, application site and the like, and the preparation is formulated by a method known to those of ordinary skill in the art.

The pharmaceutical composition of the present invention can be placed in the market as a completed liquid preparation, or as a kit including a powder or lyophilized product containing the active ingredient etc. and a liquid for dissolution. For example, the active ingredient, 2-amino-2-[2-(4-octylphenyl)ethyl]propane-1,3-diol or a pharmaceutically acceptable acid addition salt thereof (particularly hydrochloride) is dissolved in purified water and the resulting solution is sterilized by filtration, filled in vials and freeze-dried in vacuo to give lyophilized products. As a liquid for dissolution, an aqueous solution is prepared by dissolving cyclodextrin to be used in the present invention and, where necessary, saccharide, in distilled water. The aforementioned lyophilized product can be dissolved in the liquid for dissolution when in use. The liquid for dissolution is used in a 5-fold to 2000-fold amount (parts by weight) relative to the amount of 2-amino-2-[2-(4-octylphenyl)ethyl]propane-1,3-diol or a pharmaceutically acceptable acid addition salt thereof. As used herein, the distilled water is preferably distilled water for injection in the case of an injection. The aforementioned lyophilized product is generally filled in vials, and after displacement with nitrogen, applied with a rubber stopper and sealed with aluminum, after which the product can be stored for a long time at room temperature. The cyclodextrin and saccharide to be further added where necessary can be contained in the lyophilized product together with the active ingredient, 2-amino-2-[2-(4-octylphenyl) ethyl]propane-1,3-diol or a pharmaceutically acceptable acid addition salt thereof, rather than in the liquid for dissolution as mentioned above. The cyclodextrin is added in an amount of 1 - 50 parts by weight, particularly preferably 10 - 30 parts by weight, per part by weight of the above-mentioned active ingredient. The saccharide to be further added where necessary is added in an amount of 1 - 100 parts by weight, particularly preferably 5 - 80 parts by weight, per part by weight of the above-mentioned active ingredient.

The pharmaceutical composition of the present invention may appropriately contain, besides the above-mentioned ingredients, solubilizer, isotonicity agent, pH adjusting agent, buffer, antioxidant, thickener, surfactant, preservative, humectant, aromatic, coloring agent and the like. These additives may be added when formulating the pharmaceutical composition of the present invention into a preparation or may be added to the liquid for dissolution in the kit preparation for dissolution when in use.

The pharmaceutical composition of the present invention can be used for the suppression of rejection after organ or bone marrow transplantation, immunosuppressive sustention therapy therefor or the treatment of the diseases of the eye such as Behçet's disease, uveitis and the like, dermatitis inclusive of psoriasis, atopic dermatitis, contact dermatitis and allergic dermatitis, and the like. More particularly, the pharmaceutical preparation of the present invention can be used for the prophylaxis and treatment of various applicable symptoms conventionally performed with oral preparations, such as for immunosuppression in organ or bone marrow transplantation, various autoimmune diseases, various allergic diseases and the like.

Accordingly, the composition of the present invention can be used as a liquid agent for the prophylaxis and treatment of resistance or rejection in organ or tissue transplantation (e.g., transplantation inclusive of allograft of heart, kidney, liver, lung, bone marrow, cornea, pancreas, small bowel, limb, muscle, nerve, fatty marrow, duodenum, skin, pancreatic islet cell etc.), graft-versus-host (GvH) disease in bone marrow or small bowel transplantation, autoimmune diseases (e.g., rheumatoid arthritis, systemic lupus erythematosus, nephrotic syndrome lupus, Hashimoto's thyroiditis, multiple sclerosis, myasthenia gravis, type I diabetes mellitus, type II adult onset diabetes mellitus, uveitis, nephrotic syndrome, stroid-dependent and steroid-resistant nephrosis, palmoplantar pustulosis, allergic encephalomyelitis, glomerulonephritis etc.), and infectious diseases caused by pathogenic microorganisms. The composition of the present invention can be also used for the treatment of the onset of inflammatory, proliferative and ultraproliferative skin diseases and immunologically-mediated diseases of the skin, such as psoriasis, psoriatic arthritis, atopic eczema (atopic dermatitis), contact dermatitis and further, eczematous dermatitis, seborrheic dermatitis, lichen planus, pemphigus, bullous permphigoid, epidermolysis bullosa, urticaria, angioedemas, vasculitides, erythema, cutaneous eosinophilias, acne, alopecia areata, eosinophilic fasciitis and atherosclerosis. The pharmaceutical composition of the present invention can be more particularly used for hair revitalizing, such as in the treatment of female or male pattern alopecia or senile alopecia by providing epilation prevention, hair germination and/or promotion of hair generation and hair growth.

The composition of the present invention can be also used for the treatment of respiratory diseases such as sarcoidosis, pulmonary fibrosis, idiopathic interstitial pneumonia and reversible obstructive airways disease including conditions such as asthma including bronchial asthma, infantile asthma, allergic asthma, intrinsic asthma, extrinsic asthma and dust asthma, particularly chronic or inveterate asthma (e.g., late asthma and airway hyperresponsiveness), and bronchitis and the like. The composition of the present invention can be also used for liver disorders associated with ischemia. Moreover, it is effective for specific eye disease such as conjunctivitis, keratoconjunctivitis, keratitis, vernal conjunctivitis, uveitis associated with Behçet's disease, herpetic keratitis, conical cornea, dystorphia epithelialis corneae, keratoleukemia, ocular pemphigus, Mooren's ulcer, scleritis, Graves' ophthalmopathy, severe intraocular inflammation and the like.

The composition of the present invention can be also used for the prophylaxis and treatment of inflammation of mucosa or blood vessels such as leukotriene B4-mediated diseases, gastric ulcer, damage of blood vessel due to ischemic diseases and thrombosis, ischemic bowel disease, inflammatory bowel diseases (e.g., Crohn's disease and ulcerative colitis) and necrotizing enterocolitis), and intestinal lesions associated with thermal burns. The inventive composition can be used for the prophylaxis and treatment of renal diseases such as interstitial nephritis, Goodpasture's syndrome, hemolytic uremic syndrome and diabetic nephropathy; neuropathy selected from multiple myositis, Guillain-Barre syndrome, Meniere's disease and radiculopathy; endocrine diseases such as hyperthyroidism and Basedow's disease; hematic diseases such as pure red cell aplasia, aplastic anemia, hypoplastic anemia, idiopathic thrombocytopenic purpura, autoimmune hemolytic anemia, agranulocytosis and anerythroplasia; bone diseases such as osteoporosis; respiratory diseases such as sarcoidosis, pulmonary fibrosis and idiopathic interstitial pneumonia; skin disease such as dermatomyositis, vitiligo vulgaris, ichthyosis vulgaris, photoallergic sensitivity and cutaneous T cell lymphoma; circulatory diseases such as arteriosclerosis, aortitis, polyarteritis nodosa and amyocardosis; collagen diseases such as scleroderma, Wegener's granulomatosis and Sjögren's syndrome; adiposis; eosinophilic fasciitis; periodontal disease; nephrotic syndrome; hemolytic uremic syndrome; and muscular dystrophy.

The inventive composition is also suitable for the prophylaxis and treatment of inflammation/allergy of intestine, such as Coeliac disease, proctitis, eosinophilic gastroenteritis, mastocytosis, Crohn's disease, ulcerative colitis; and allergic diseases associated with food, which shows symptoms directly irrelevant to gastrointestinal tract, such as migraine, rhinitis and eczema.

2-Amino-2-[2-(4-octylphenyl)ethyl]propane-1,3-diol and a pharmaceutically acceptable acid addition salt thereof, which are the active ingredients of the pharmaceutical composition of the present invention, have liver regenerating activity and/or activity of promoting hypertrophy and hyperplasia of hepatocytes. Therefore, the inventive composition can be used for the prophylaxis and treatment of liver diseases such as immunogenic diseases (e.g., chronic autoimmune liver diseases including autoimmune hepatitis, primary biliary cirrhosis and sclerosing cholangitis), partial hepatectomy, acute liver necrosis (e.g., necrosis caused by toxins, viral hepatitis, shock or anoxia), B=virus hepatitis, non-A/non-B hepatitis and cirrhosis.

The composition of the present invention can be also used as a composition for antibacterial agent, and therefore, can be used for the treatment of the diseases caused by pathogenic microorganisms. Moreover, the inventive composition can be used for the prophylaxis and treatment of malignant rheumatoid arthritis, amyloidosis, fulminant hepatitis, Shy-Drager syndrome, pustular psoriasis, Behçet's disease, systemic lupus erythematosus, endocrine ophthalmopathy, progressive systemic sclerosis, mixed connective tissue disease, aortitis syndrome, Wegener's granulomatosis, active chronic hepatitis, Evans syndrome, pollinosis, idiopathic hypoparathyroidism, Addison disease (autoimmune adrenalitis), autoimmune orchitis, autoimmune oophoritis, cold hemagglutinin disease, paroxysmal cold hemoglobinuria, pernicious anemia, adult T cell leukemia, autoimmune atrophic gastritis, lupoid hepatitis, tubulointerstitial nephritis, membranous nephritis, amyotrophic lateral sclerosis, rheumatic fever, postmyocardial infarction syndrome and sympathetic ophthalmitis.

Where the case demands, the composition of the present invention can be used concurrently with other immunosuppressant (s), steroid (s) (e.g., prednisolone, methyl prednisolone, dexamethasone, hydrocortisone etc.) or nonsteroidal antiinflammatory agents. Particularly preferable other immunosuppressants is selected from azathioprine, brequinar sodium, deoxyspergualin, mizoribine, mycophenolic acid 2-morpholinoethyl ester, cyclosporine, rapamycin, tacrolimus monohydrate, leflunomide and OKT-3.

Though subject to change depending on the disease to be treated, conditions thereof, sex and age of patient, application site and the like, the composition of the present invention containing the active ingredient of the present compound and the like in a proportion of 0.00001 - 20 wt%, preferably 0.0001 - 10 wt%, can be applied one to several times (e.g., 2-5 times) a day to achieve a clinically preferable effect.

### Best Mode to Practice the Invention

The present invention is explained in more detail in the following by referring to Examples, Comparative Examples and Experimental Examples.

In the following examples, any proportion is based on weight and shows w/v%, unless otherwise specified. In the examples, the present compound means 2-amino-2-[2-(4-octylphenyl)ethyl]propane-1,3-diol hydrochloride, as mentioned above.

### Example 1

An injection containing the present compound, which has the following formulation, is produced.

| | |
|---|---|
| present compound | 0.1% |
| α-cyclodextrin (trademark: Cerdex A-100) | 1.0% |
| D-mannitol | 5.0% |

The above composition is dissolved in distilled water for injection to give an injection having the total amount of 10 ml. Where necessary, typical additives, such as preservative, can be incorporated.

### Example 2

An injection containing the present compound, which has the following formulation, is produced.

| | |
|---|---|
| present compound | 0.1% |
| β-cyclodextrin partially etherified with 2-hydroxypropyl (trademark: Cerdex HP-β-CD) | 1.0% |
| D-mannitol | 5.0% |

The above composition is dissolved in distilled water for injection to give an injection having the total amount of 10 ml. Where necessary, typical additives, such as preservative, can be incorporated.

### Example 3

An injection containing the present compound, which has the following formulation, is produced.

| | |
|---|---|
| present compound | 0.1% |
| α-cyclodextrin (trademark: Cerdex A-100) | 1.0% |

The above composition is dissolved in distilled water for injection containing typical additives, such as preservative, where necessary. After sterilization by filtration, the total amount of 10 ml is packed in a vial, which is lyophilized by a conventional method to give an injection.

### Example 4

An injection containing the present compound, which has the following formulation, is produced.

| | |
|---|---|
| present compound | 0.1% |
| β-cyclodextrin partially etherified with 2-hydroxypropyl (trademark: Cerdex HP-β-CD) | 1.0% |

The above composition is dissolved in distilled water for injection containing typical additives, such as preservative, where necessary. After sterilization by filtration, the total amount of 10 ml is packed in a vial, which is lyophilized by a conventional method to give an injection.

### Example 5

An injection containing the present compound, which has the following formulation, is produced.

| | |
|---|---|
| present compound | 0.1% |
| β-cyclodextrin partially etherified with 2-hydroxypropyl (trademark: Cerdex HP-β-CD) | 2.0% |
| sodium chloride | 0.9% |

The above composition is dissolved in distilled water for injection to give an injection having the total amount of 10 ml. Where necessary, typical additives, such as preservative, can be incorporated.

### Example 6

An eye drop containing the present compound, which has the following formulation, is produced.

| | |
|---|---|
| present compound | 0.1% |
| α-cyclodextrin (trademark: Cerdex A-100) | 1.0% |
| D-mannitol | 5.0% |

The above composition is dissolved in sterile purified water to give an eye drop having the total amount of 10 ml. Where necessary, typical additives, such as preservative, can be incorporated.

### Example 7

An eye drop containing the present compound, which has the following formulation, is produced.

| | |
|---|---|
| present compound | 0.1% |
| β-cyclodextrin partially etherified with 2-hydroxypropyl (trademark: Cerdex HP-β-CD) | 1.0% |
| D-mannitol | 5.0% |

The above composition is dissolved in sterile purified water to give an eye drop having the total amount of 10 ml. Where necessary, typical additives, such as preservative, can be incorporated.

### Comparative Example 1

| | |
|---|---|
| present compound | 0.1% |

The present compound is dissolved in distilled water for injection to give an injection having the total amount of 10 ml.

### Comparative Example 2

| | |
|---|---|
| present compound | 0.1% |
| sodium chloride | 0.9% |

The above composition is dissolved in distilled water for injection to give an injection having the total amount of 10 ml.

### Comparative Example 3

| | |
|---|---|
| present compound | 0.1% |
| D- mannitol | 5.0% |

The above composition is dissolved in distilled water for injection to give an injection having the total amount of 10 ml.

### Comparative Example 4

| | |
|---|---|
| present compound | 0.1% |
| D-mannitol | 5.0% |
| sodium laurylsulfate | 1.0% |

The above composition is dissolved in distilled water for injection to give an injection having the total amount of 10 ml.

### Comparative Example 5

| | |
|---|---|
| present compound | 0.1% |
| D-mannitol | 5.0% |
| polysorbate 80 | 1.0% |

The above composition is dissolved in distilled water for injection to give an injection having the total amount of 10 ml.

### Comparative Example 6

| | |
|---|---|
| present compound | 0.1% |
| D-mannitol hydrogenated polyoxyethylene | 5.0% |
| castor oil 60 (HCO-60) | 1.0% |

The above composition is dissolved in distilled water for injection to give an injection having the total amount of 10 ml.

### Comparative Example 7

| | |
|---|---|
| present compound | 0.1% |
| D- mannitol | 5.0% |
| polyvinylpyrrolidone 12 PF | 1.0% |

The above composition is dissolved in distilled water for injection to give an injection having the total amount of 10 ml.

### Experimental Example 1: Hemolysis test

The preparations obtained in Examples 1 and 2 were prepared into sample solutions according to "Yakuan No. 2" (Test method for topical disorder caused by injection (draft), January 12, 1979, Safety Section, Pharmaceutical Affairs Bureau, Ministry of Health and Welfare) and the absorbance at 540 nm was measured according to the method of Inglot et al., Biochem. Pharmacol. Vol. 17, p. 269 (1968). As a result, the preparations of Examples 1 and 2 were found to have significantly lower hemolytic property. In contrast, the preparations of comparative Examples 1-7 showed hemolytic property.

### Experimental Example 2: Local irritation test

The preparation obtained in Example 1 was intravenously repeat administered to 5-week-old LEW rats for 5 consecutive days and local irritation was confirmed using swelling ratio of the tail {(diameter of tail of drug administration group - diameter of tail of control) ÷ diameter of tail of control × 100} as an index. As a result, the preparation of Example 1 achieved 0%, showing presence of no local irritation.

### Experimental Example 3: Crystal precipitation test

The preparations obtained in Examples 1 and 2 were sterilized by filtration, filled in ampoules, melt-sealed and sterilized by heating at 121°C for 20 minutes to give injections having the total amount of 2 ml. These preparations were left standing at room temperature for 1 week. As a result, the preparations of Examples 1 and 2 showed no precipitation of crystals. The preparations of Examples 1 and 2 were left standing in a refrigerator for 1 week. Neither preparation showed precipitation of crystals. In contrast, the preparations of Comparative Examples 1, 2, 3, 4 and 7 showed precipitation of crystals both at room temperature and in cold storage.

### Experimental Example 4: Foaming test

The ampoules were stood erected, laid down and then stood erected, which steps were taken as one cycle. This cycle was repeated 10 times for the preparations obtained in Examples 1 and 2. The generated foams disappeared within 1 minute in the case of the preparations obtained in Examples 1 and 2, demonstrating a remarkably short defoaming time. In contrast, the generated foams did not disappear for at least 5 minutes in the case of the preparations of Comparative Examples 1-7.

### Industrial Applicability

A pharmaceutical composition containing 2-amino-2-[2-(4-octylphenyl) ethyl]propane-1,3-diol or a pharmaceutically acceptable acid addition salt thereof, which is suitable for a liquid agent, which can be readily prepared into a pharmaceutical agent, which shows less side effects, such as hemolysis, less local irritation and superior defoaming performance, and which inhibits crystal precipitation of the active ingredient compound, can be provided by the addition of cyclodextrin as a stabilizer to 2-amino-2-[2-(4-octylphenyl) ethyl]propane-1,3-diol or a pharmaceutically acceptable acid addition salt thereof. Incorporation of saccharide into this composition results in an even more reduced local irritation.

## Claims

1. A pharmaceutical composition comprising 2-amino-2-[2-(4-octylphenyl)ethyl]propane-1,3-diol or a pharmaceutically acceptable acid addition salt thereof as an active ingredient, and cyclodextrin as a stabilizer.

2. The pharmaceutical composition of claim 1, wherein the cyclodextrin is naturally occurring cyclodextrin, branched cyclodextrin, alkyl cyclodextrin or hydroxyalkyl cyclodextrin.

3. The pharmaceutical composition of claim 1, wherein the cyclodextrin is incorporated in an amount of 1 - 50 parts by weight per part by weight of 2-amino-2-[2-(4-octylphenyl)ethyl]propane-1,3-diol or a pharmaceutically acceptable acid addition salt thereof.

4. The pharmaceutical composition of claim 1, further comprising saccharide.

5. The pharmaceutical composition of claim 4, wherein the saccharide is selected from monosaccharides, disaccharides and sugar alcohol.

6. The pharmaceutical composition of claim 4 or claim 5, wherein the saccharide is one or more kinds selected from D-mannitol, glucose, D-xylitol, D-maltose, D-sorbitol, lactose, fructose and sucrose.

7. The pharmaceutical composition of claim 4, wherein the saccharide is contained in an amount of 1 - 100 parts by weight per part by weight of 2-amino-2-[2-(4-octylphenyl)ethyl]propane-1,3-diol or a pharmaceutically acceptable acid addition salt thereof.

8. The pharmaceutical composition of claim 1, wherein the active ingredient is inhibited from precipitating out as crystals.

9. The pharmaceutical composition of claim 1 or claim 8, wherein the active ingredient is contained in a proportion of 0.05 - 0.1 wt% of the composition and the cyclodextrin is contained in a proportion of 1 - 50 parts by weight per part by weight of the active ingredient.

10. A kit comprising a lyophilized product of 2-amino-2-[2-(4-octylphenyl)ethyl]propane-1,3-diol or a pharmaceutically acceptable acid addition salt thereof, and a liquid for dissolution comprising an aqueous solution containing cyclodextrin.

11. The kit of claim 10 comprising a lyophilized product comprising 2-amino-2-[2-(4-octylphenyl)ethyl]propane-1,3-diol or a pharmaceutically acceptable acid addition salt thereof and cyclodextrin, and a liquid for dissolution comprising distilled water.

12. The kit of claim 10 or claim 11, wherein the cyclodextrin is contained in an amount of 1 - 50 parts by weight per part by weight of 2-amino-2-[2-(4-octylphenyl)ethyl]propane-1,3-diol or a pharmaceutically acceptable acid addition salt thereof.

13. The kit of claim 10 or claim 11, wherein one or both of the lyophilized product and the liquid for dissolution comprise(s) saccharide.

14. The kit of claim 13, wherein the saccharide is contained in an amount of 1 - 100 parts by weight per part by weight of 2-amino-2-[2-(4-octylphenyl)ethyl]propane-1,3-diol or a pharmaceutically acceptable acid addition salt thereof.

## Patentansprüche

1. Pharmazeutische Zusammensetzung, die 2-Amino-2-[2-(4-octylphenyl)-ethyl]propan-1,3-diol oder ein pharmazeutisch annehmbares Säureadditionssalz davon als Wirkstoff und Cyclodextrin als Stabilisator umfasst.

2. Pharmazeutische Zusammensetzung gemäß Anspruch 1, wobei das Cyclodextrin natürlich vorkommendes Cyclodextrin, verzweigtes Cyclodextrin, Alkylcyclodextrin oder Hydroxyalkylcyclodextrin ist.

3. Pharmazeutische Zusammensetzung gemäß Anspruch 1, wobei das Cyclodextrin in einer Menge von 1 bis 50 Gewichtsteilen pro Gewichtsteil 2-Amino-2-[2-(4-octylphenyl)ethyl]propan-1,3-diol oder des pharmazeutisch annehmbaren Säureadditionssalzes davon eingearbeitet wird.

4. Pharmazeutische Zusammensetzung gemäß Anspruch 1, die weiterhin Saccharid umfasst.

5. Pharmazeutische Zusammensetzung gemäß Anspruch 4, wobei das Saccharid aus Monosacchariden, Disacchariden und Zuckeralkohol ausgewählt ist.

6. Pharmazeutische Zusammensetzung gemäß Anspruch 4 oder 5, wobei es sich bei dem Saccharid um eine oder mehrere Arten handelt, die aus D-Mannit, Glucose, D-Xylit, D-Maltose, D-Sorbit, Lactose, Fructose und Saccharose ausgewählt sind.

7. Pharmazeutische Zusammensetzung gemäß Anspruch 4, wobei das Saccharid in einer Menge von 1 bis 100 Gewichtsteilen pro Gewichtsteil 2-Amino-2-[2-(4-octylphenyl)ethyl]propan-1,3-diol oder des pharmazeutisch annehmbaren Säureadditionssalzes davon enthalten ist.

8. Pharmazeutische Zusammensetzung gemäß Anspruch 1, wobei das Ausfallen des Wirkstoffs in Form von Kristallen gehemmt ist.

9. Pharmazeutische Zusammensetzung gemäß Anspruch 1 oder 8, wobei der Wirkstoff in einem Anteil von 0,05 bis 0,1 Gew.-% der Zusammensetzung enthalten ist und das Cyclodextrin in einem Anteil von 1 bis 50 Gewichtsteilen pro Gewichtsteil des Wirkstoffs enthalten ist.

10. Kit, der ein lyophilisiertes Produkt von 2-Amino-2-[2-(4-octylphenyl)-ethyl]propan-1,3-diol oder eines pharmazeutisch annehmbaren Säureadditionssalzes davon und eine Flüssigkeit zur Auflösung, die eine Cyclodextrin enthaltende wässrige Lösung umfasst, umfasst.

11. Kit gemäß Anspruch 10, der ein lyophilisiertes Produkt, das 2-Amino-2-[2-(4-octylphenyl)ethyl]propan-1,3-diol oder ein pharmazeutisch annehmbares Säureadditionssalz davon und Cyclodextrin umfasst, und eine Flüssigkeit zur Auflösung, die destilliertes Wasser umfasst, umfasst.

12. Kit gemäß Anspruch 10 oder 11, wobei das Cyclodextrin in einer Menge von 1 bis 50 Gewichtsteilen pro Gewichtsteil 2-Amino-2-[2-(4-octylphenyl)ethyl]propan-1,3-diol oder des pharmazeutisch annehmbaren Säureadditionssalzes davon enthalten ist.

13. Kit gemäß Anspruch 10 oder 11, wobei das lyophilisierte Produkt und/oder die Flüssigkeit zur Auflösung Saccharid umfasst.

14. Kit gemäß Anspruch 13, wobei das Saccharid in einer Menge von 1 bis 100 Gewichtsteilen pro Gewichtsteil 2-Amino-2-[2-(4-octylphenyl)ethyl]propan-1,3-diol oder des pharmazeutisch annehmbaren Säureadditionssalzes davon enthalten ist.

## Revendications

1. Composition pharmaceutique comprenant un 2-amino-2-[2-(4-octylphényl)éthyl]propane-1,3-diol ou un sel d'addition acide, pharmaceutiquement acceptable de celui-ci en tant qu'ingrédient actif, et de la cyclodextrine en tant que stabilisant.

2. Composition pharmaceutique selon la revendication 1, dans laquelle la cyclodextrine est de la cyclodextrine présente dans la nature, de la cyclodextrine ramifiée, de l'alkylcyclodextrine ou de l'hydroxyalkyl-cyclodextrine.

3. Composition pharmaceutique selon la revendication 1, dans laquelle la cyclodextrine est incorporée en une quantité de 1 à 50 parties en poids par partie en poids de 2-amino-2-[2-(4-octylphényl)-éthyl]-propane-1,3-diol ou d'un sel d'addition acide, pharmaceutiquement acceptable de celui-ci.

4. Composition pharmaceutique selon la revendication 1 comprenant, en outre, du saccharide.

5. Composition pharmaceutique selon la revendication 4, dans laquelle le saccharide est choisi parmi les monosaccharides, les disaccharides et du sucre-alcool.

6. Composition pharmaceutique selon la revendication 4 ou la revendication 5, dans laquelle le saccharide est d'un ou de plusieurs types choisis parmi le D-mannitol, le glucose, le D-xylitol, le D-maltose, le D-sorbitol, le lactose, le fructose et le saccharose.

7. Composition pharmaceutique selon la revendication 4, dans laquelle le saccharide est contenu en une quantité de 1 à 100 parties en poids par partie en poids de 2-amino-2-[2-(4-octylphényl)-éthyl]-propane-1,3-diol ou d'un sel d'addition acide, pharmaceutiquement acceptable de celui-ci.

8. Composition pharmaceutique selon la revendication 1, dans laquelle l'ingrédient actif est empêchée de précipiter sous forme de cristaux.

9. Composition pharmaceutique selon la revendication 1 ou la revendication 8, dans laquelle l'ingrédient actif est contenu en une proportion de 0,05 à 0,1 % en poids de la composition et la cyclodextrine est contenue en une proportion de 1 à 50 parties en poids par partie en poids de l'ingrédient actif.

10. Kit comprenant un produit lyophilisé de 2-amino-2-[2-(4-octylphényl)éthyl]propane-1,3-diol ou d'un sel d'addition acide, pharmaceutiquement acceptable de celui-ci, et un liquide pour la dissolution comprenant une solution aqueuse contenant de la cyclodextrine.

11. Kit selon la revendication 10 comprenant un produit lyophilisé comprenant du 2-amino-2-[2-(4-octylphényl)-éthyl]-propane-1,3-diol ou d'un sel d'addition acide, pharmaceutiquement acceptable de celui-ci et de la cyclodextrine, et un liquide pour la dissolution comprenant de l'eau distillée.

12. Kit selon la revendication 10 ou la revendication 11, dans laquelle la cyclodextrine est contenue en une quantité de 1 à 50 parties en poids par partie en poids de 2-amino-2-[2-(4-octylphényl)éthyl]-propane-1,3-diol ou d'un sel d'addition acide, pharmaceutiquement acceptable de celui-ci.

13. Kit selon la revendication 10 ou la revendication 11, dans laquelle le produit lyophilisé ou le liquide pour la dissolution, ou les deux, comprend/comprennent du saccharide.

14. Kit selon la revendication 13, dans laquelle le saccharide est contenu en une quantité de 1 à 100 parties en poids par partie en poids de 2-amino-2-[2-(4-octylphényl)éthyl]-propane-1,3-diol ou d'un sel d'addition acide, pharmaceutiquement acceptable de celui-ci.
